(19) Europäisches Patentamt
European Patent Office
Office européen des brevets

(11) **EP 4 114 487 B1**

(12) **EUROPÄISCHE PATENTSCHRIFT**

(45) Veröffentlichungstag und Bekanntmachung des Hinweises auf die Patenterteilung:
**17.04.2024 Patentblatt 2024/16**

(21) Anmeldenummer: **21706898.0**

(22) Anmeldetag: **18.02.2021**

(51) Internationale Patentklassifikation (IPC):
**A61M 16/04** *(2006.01)* **A61M 16/00** *(2006.01)*

(52) Gemeinsame Patentklassifikation (CPC):
**A61M 16/044;** A61M 2016/0027; A61M 2205/0238; A61M 2205/3344; A61M 2205/7527

(86) Internationale Anmeldenummer:
**PCT/EP2021/053948**

(87) Internationale Veröffentlichungsnummer:
**WO 2021/175602 (10.09.2021 Gazette 2021/36)**

(54) **TRACHEALBEATMUNGSVORRICHTUNG MIT OPTIMIERTEM CUFF-SCHLAUCH**

TRACHEAL VENTILATION DEVICE HAVING OPTIMIZED CUFF TUBE

DISPOSITIF DE VENTILATION TRACHÉALE AYANT UN TUBE À MANCHON OPTIMISÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.03.2020 DE 102020105522**

(43) Veröffentlichungstag der Anmeldung:
**11.01.2023 Patentblatt 2023/02**

(73) Patentinhaber: **Coloplast A/S**
**3050 Humlebaek (DK)**

(72) Erfinder: **SCHNELL, Ralf**
**63500 Seligenstadt (DE)**

(56) Entgegenhaltungen:
**US-A1- 2008 078 406 US-A1- 2011 027 334**

**Beschreibung**

Die vorliegende Erfindung betrifft eine Trachealbeatmungsvorrichtung, insbesondere einen Endotrachealtubus oder Tracheotomietubus, wobei die Trachealbeatmungsvorrichtung ein Kanülenrohr und eine um das Kanülenrohr herum angeordnete aufblasbare Manschette aufweist, wobei die Manschette mit einem Füllschlauch in Fluidkontakt ist. Insbesondere betrifft die Erfindung Einrichtungen und Maßnahmen zur Verbesserung der Druckmessung in der Manschette einer Trachealbeatmungsvorrichtung der vorgenannten Art.

Viele Trachealbeatmungsvorrichtungen sind mit einer aufblasbaren Manschette, dem sogenannten Cuff ausgestattet. Dabei handelt es sich im Wesentlichen um einen aufblasbaren Abdichtballon, der sich auf der distalen Seite also kurz vor dem Patientenende des Tubus befindet. Zum Befüllen des Cuffs wird über ein Füllventil Luft eingebracht. Diese gelangt über den Füllschlauch in den Cuff, und zur Einbringung der Luft kann beispielsweise ein dafür vorgesehenes Handmanometer verwendet werden, das über eine Aufblasfunktion verfügt.

Der Cuff hat die Aufgabe die Luftröhre um den Tubus herum abzudichten. Durch diese abdichtende Wirkung des Cuffs wird zum einen sichergestellt, dass bei künstlich beatmeten Patienten die von einem Beatmungsgerät zur Verfügung gestellte Beatmungsluft vollständig in die Bronchien gelangt und von dort den Patienten auch wieder durch den Tubus verlässt. Würde der Cuff nicht ausreichend abdichten, könnten signifikante Mengen Beatmungsluft durch die natürlichen Atemwege entweichen. Zum anderen verhindert die Abdichtung durch den Cuff, dass Sekret aus dem subglottischen Bereich in die Bronchien gelangt, was sonst zu aspirationsassoziierten Pneumonien führen könnte.

Die meisten Trachealbeatmungsvorrichtungen der hier beschriebenen Art sind mit sogenannten "High Volume Low Pressure Cuffs (HVLP)" ausgestattet. Im Unterschied zu einem dehnbaren Luftballon ist es bei HVLP-Cuffs nicht vorgesehen, dass sich das Material des Cuffs beim Aufblasen aufdehnt. Die Abdichtung in der Trachea wird vielmehr dadurch erreicht, dass der aufgeblasene Cuff im freien Zustand einen deutlich größeren Durchmesser (ca. 30%) aufweist als die vorgesehene Luftröhre. Bei der vorgesehenen Anwendung schmiegt sich der mit Luft gefüllte Cuff unter Ausbildung von Falten an die Konturen der Trachea an.

Diese Art von Cuff hat den Vorteil, dass der messbare Cuffdruck exakt dem Druck entspricht mit dem der Cuff auf die Trachea drückt. Damit trotz Falten eine ausreichende Abdichtung stattfindet, sollte ein handelsüblicher Cuff mit circa 15-30 mbar befüllt werden. Um bereits bei möglichst geringen Cuffdrücken ausreichende Abdichtungen zu erzielen, werden Cuffs mit möglichst dünner Wandstärke angestrebt. Zur Vermeidung von Trachealschäden sollte andererseits vermieden werden, dass der Cuffdruck in den Bereich des kapillären Perfusionsdruck ansteigt. Deshalb sollte der Cuffdruck möglichst nicht höher als 30 mbar eingestellt werden.

Aus den oben genannten Gründen muss man den Cuffdruck sehr exakt einstellen können, um einerseits die angestrebte Abdichtung zu erzielen und andererseits Trachealschädigungen zu vermeiden. Aus diesem Grund ist es üblich den Cuffdruck regelmäßig zu kontrollieren (z. B. alle 4 Stunden) bzw. durch ein geeignetes Gerät kontinuierlich zu regeln.

Nicht selten kommt es vor, dass sich im Cuff-Inneren Kondenswasser ansammelt. Es handelt sich dabei um Wasser, dass durch die Cuffwand in das Innere des Cuffs diffundiert ist. Dies kann bei der weiteren Anwendung dann problematisch werden, wenn bei der Cuffdruckkontrolle oder der Nacheinstellung des Cuffdrucks oder auch bei einer vorübergehenden beabsichtigten Entspannung des Cuffs Wasser aus dem Cuff in den Füllschlauch gelangt. Dies kann dann nämlich dazu führen, dass bei der Cuffdruckkontrolle Artefakte auftreten, da die Verbindung zwischen Messgerät und Cuff durch das Wasser im Füllschlauch gestört wird.

Artefakte können insbesondere dann auftreten, wenn das Wasser im Füllschlauch durch viele kleine Luftbläschen unterbrochen wird. In diesen Fällen kann es vorkommen, dass der eingestellte Druck im Kontrollballon nicht ausreicht, um das Wasser im Füllschlauch in den Cuff zu drücken. Dies bedeutet, dass im Cuff keine Luft ankommt und somit auch kein Druck aufgebaut wird, obwohl ein am Kontrollballon angeschlossenes Manometer den gewünschten Druck anzeigt.

Um das Risiko für Wasserbildung im Cuff zu reduzieren, werden teilweise Cuffs mit dickeren Wandstärken verwendet. Cuffs mit dicken Wandstärken dichten jedoch nicht so gut gegen Aspiration ab wie dünne Cuffs. Auch die Verwendung von Materialien, die sehr gute Barriereeigenschaften gegenüber Wasser haben, wie z.B. Polypropylen, Polyethylen, Polyester oder Teflon, käme in Betracht. Diese Materialien lassen sich jedoch nicht so gut verkleben, weshalb die meisten Hersteller von Endotrachealtuben, Tracheotomietuben und anderen Trachealbeatmungsvorrichtungen mit HVLP-Cuffs PVC oder Polyurethan verwenden. Allerdings sind die Wasserbarriereeigenschaften von Polyurethan recht ungünstig und so sammelt sich insbesondere bei dünnen Polyurethancuffs sehr schnell Wasser an. Die Barriereeigenschaften von PVC sind im Vergleich zu Polyurethan zwar deutlich höher, aber sogar bei PVC-Cuffs mit Wandstärken von 90 μm wurde Wasser im Cuff beobachtet.

Um die Ansammlung von Kondenswasser im Cuff zu verhindern wird in EP 1 861 152 B1 ein Cuff mit wasserundurchlässiger Beschichtung beschrieben. Solch eine Beschichtung kann jedoch die Flexibilität des Cuffs reduzieren und somit seine Abdichtleistung gegenüber Sekret verringern. Alternativ könnte auch der Innendurchmesser des Füllschlauchs deutlich größer ausgeführt sein, als üblich, um die freie Gaspassage beim Messen des Drucks im Cuff zu gewährleisten. Allerdings hätte dies zur Folge, dass die Wandstärke der Kanülen erhöht werden müsste, damit der Füllschlauch mit vergrößertem Innendurchmesser darin Platz findet, was auf Kosten des Querschnitts des Lumens des Kanülenrohrs gehen würde.

Folgende Dokumente US 2008/078406 A1 und US 2011/027334 A1 offenbaren weitere Trachealbeatmungsvorrichtungen mit Kanülenrohr und Füllschlauch.

Es ist daher die Aufgabe der vorliegenden Erfindung eine neue technische Lösung bereitzustellen, mit der das Risiko, dass bei der Cuffdruckmessung aufgrund von Wasseransammlungen im Cuff Artefakte auftreten, verringert werden kann.

Diese Aufgabe wird erfindungsgemäß gelöst durch eine Trachealbeatmungsvorrichtung gemäß Anspruch 1. Die abhängigen Ansprüchen definieren besondere Ausführungsformen der Erfindung.

Die erfindungsgemäß beanspruchte Trachealbeatmungsvorrichtung ist zum Einführen in die Trachea geeignet, um darüber die Atmung zu ermöglichen. Hierfür weist die Trachealbeatmungsvorrichtung ein Kanülenrohr zum Einführen in die Luftröhre auf, dessen Rohrwand sich zwischen der Rohröffnung am proximalen Rohrende und der Rohröffnung am distalen Rohrende erstreckt und ein Lumen definiert, durch das in dem in die Trachea eingeführten Zustand Atemluft bzw. Atemgas strömen kann. Bei bestimmten Ausführungsformen handelt es sich bei der Trachealbeatmungsvorrichtung um einen Endotrachealtubus, welcher durch den Larynx in die Trachea geführt wird, bei anderen Ausführungsformen handelt es sich um einen Tracheotomietubus, der auch häufig als Tracheostomiekanüle bezeichnet wird. Dieser Tubus wird durch ein am Hals befindliches Stoma in die Trachea geführt.

Die Begriffe "distal" und "proximal" werden im Sinne der vorliegenden Erfindung aus der Sicht eines die Trachealbeatmungsvorrichtung anwendenden Arztes verwendet, d.h. das proximale Ende der Trachealbeatmungsvorrichtung ist das Ende, welches nach dem Einführen in die Trachea außerhalb des Patientenkörpers verbleibt, während das distale Ende der Trachealbeatmungsvorrichtung in die Trachea des Patienten eingeführt wird.

Die aufblasbare Manschette an der Außenwand des Kanülenrohres (Cuff), welche in dem in die Trachea eingeführten Zustand der gas- und flüssigkeitsdichten Abdichtung der Außenwand des Kanülenrohres gegenüber der Innenwand der Luftröhre dient, ist vorzugsweise ringförmig um das Kanülenrohr angeordnet und ist an der Anlagefläche rundum fluiddicht mit der Außerwand des Kanülenrohrs verbunden.

Der Füllschlauch ist ein Schlauch dessen Schlauchwand sich zwischen der Schlauchöffnung am proximalen Schlauchende und der Schlauchöffnung, die am distalen Schlauchende in der aufblasbaren Manschette mündet, erstreckt und ein Lumen definiert, durch das in dem in die Trachea eingeführten Zustand Luft oder ein anderes Gas in die Manschette geblasen werden kann oder Luft aus der Manschette abgelassen werden kann. Der Füllschlauch kann entweder in der Rohrwand des Kanülenrohres geführt sein oder entlang der Innenwand oder entlang der Außenwand des Kanülenrohres geführt sein.

Die äußere Schicht des Füllschlauchs besteht aus einem elastischen Schlauchmaterial. Typischerweise, handelt es sich hierbei um ein Elastomer, das beispielsweise ausgewählt sein kann unter Polyvinylchlorid, Polyurethan, Polyethylen, Polypropylen, Silikon, Ethylen-Vinylacetat-Copolymer.

Die erfindungsgemäß vorgeschlagene hydrophile Schicht ist auf die Innenseite der äußeren Schlauchschicht des Füllschlauchs aufgeschichtet und bildet die Grenzschicht zwischen Schlauchwand und Schlauchlumen. Bei manchen Ausführungsformen der Erfindung besteht die äußere Schlauchschicht aus mehr als einem Material, beispielsweise aus einer ersten tragenden Schicht, die auf der inneren Seite des äußeren Füllschlauchschicht angeordnet ist, und einer zweiten Abschluss- oder Deckschicht, die auf der äußeren Seite der äußeren Füllschlauchschicht angeordnet ist.

Unter dem Begriff "Schicht" ist hier eine feste Schicht, d.h. nicht flüssige Schicht, zu verstehen. Die erfindungsgemäß vorgeschlagene hydrophile Schicht überzieht die innere Oberfläche der äußeren Füllschlauchschicht flächig und ist fest mit dieser verbunden.

Bei bestimmten Ausführungsformen kann zwischen der äußeren Schlauchschicht und der inneren hydrophilen Schicht eine Verbindungsschicht als Haftvermittler vorgesehen, um eine besonders gute Verbindung zwischen der äußeren Schlauchschicht und der inneren hydrophilen Schicht zu erzielen.

Durch die feste Verbindung zwischen hydrophiler Schicht und der inneren Oberfläche der äußeren Füllschlauchschicht, wahlweise über eine dazwischen angeordnete Verbindungsschicht (Haftvermittler), kann die hydrophile Schicht durch im Füllschlauch auftretendes Wasser nicht gelöst und auch nicht abgelöst werden, sondern verbleibt über die gesamte Dauer der Verwendung des Trachealbeatmungsvorrichtung durchgängig fest mit der äußeren Füllschlauchschicht verbunden.

Unter dem Begriff "hydrophile" Schicht ist hier insbesondere eine Schicht zu verstehen, die aus einem hydrophilen Schichtmaterial besteht, das gemessen nach der Methode des liegenden Tropfens und berechnet nach der Youngschen Gleichung

$$\cos \Theta = \frac{\sigma_{SG} - \sigma_{LS}}{\sigma_{LG}}$$

mit destilliertem Wasser bei 20°C einen Kontaktwinkel $\theta$ < 80° ausbildet. Gemessen wird der Kontaktwinkel auf einer ebenen Musterfläche des für die hydrophile Schicht verwendeten Schichtmaterials.

Bestimmte Ausführungsformen der Erfindung sind dadurch gekennzeichnet, dass die hydrophile Schicht aus einem Material besteht, das unter den genannten Bedingungen mit Wasser einen Kontaktwinkel θ ≤ 70° ausbildet. Bei speziellen Ausführungsformen der Erfindung besteht die hydrophile Schicht aus einem Material, das unter den genannten Bedingungen mit Wasser einen Kontaktwinkel θ ≤ 60° oder gar θ ≤ 50°ausbildet.

Die erfindungsgemäß vorgeschlagene hydrophile Beschichtung bewirkt, dass sich im Füllschlauch befindliches Wasser auch bereits bei in Relation zum vorgesehenen Cuffdruck kleinen Druckdifferenzen in beide Richtungen bewegen lässt. Auf diese Weise wird sichergestellt, dass ein Druckausgleich zwischen Manometer und Cuff erfolgt. Die vom Erfinder der vorliegenden Anmeldung durchgeführten Versuche haben gezeigt, dass die Druckdifferenz, die notwendig war, um Wasser durch einen Füllschlauch zu drücken durch die hydrophile Beschichtung im Vergleich zu einem nicht mit einer hydrophilen Schicht ausgerüsteten Füllschlauch um bis zu Faktor 5 reduziert werden kann.

Erfindungsgemäß besteht die hydrophile Schicht aus einem Material, das unter den genannten Bedingungen mit Wasser einen geringeren Kontaktwinkel θ ausbildet als die oben genannten herkömmlichen Füllschlauchmaterialien. Bei speziellen Ausführungsformen der Erfindung besteht die hydrophile Schicht aus einem Material, dessen Kontaktwinkel θ um wenigstens 15°, vorzugsweise wenigstens 20°, noch bevorzugter wenigstens 25° geringer ist als der Kontaktwinkel des für die äußere Schlauchschicht verwendeten herkömmlichen Füllschlauchmaterials.

Grundsätzlich kann die hydrophile Schicht aus jedem hydrophilen Material bestehen, das für die Ausbildung einer inneren Beschichtung in einem Füllschlauch geeignet ist und das unter den genannten Bedingungen mit Wasser den verlangten Kontaktwinkel ausbildet. Beispiele für hydrophile Materialien, die hierfür geeignet sind, sind ausgewählt unter hydrophilen Poly(lactamen), Polyurethanen, Polyvinylalkohol, Polyvinylethern, Copolymeren auf Maleinsäureanhydridbasis, Vinylaminen, Polyethyleniminen, Polyethylenoxiden, Polypropylenoxiden, Poly(carbonsäuren), Polyanhydriden, Polyphosphazenen, Polypeptiden, Polysacchariden und Oligonucleotiden, die unter den genannten Bedingungen mit Wasser den verlangten Kontaktwinkel ausbilden.

Bei bestimmten Ausführungsformen ist das für die Ausbildung der hydrophilen Beschichtung verwendete hydrophile Material ausgewählt unter Polyvinylpyrrolidon, Polyvinylpyrrolidon-Copolymer, Polyvinylpolypyrrolidon, Polylactiden, Polyglycoliden und Polycaprolactonen, die unter den genannten Bedingungen mit Wasser den verlangten Kontaktwinkel ausbilden.

Bei speziellen Ausführungsformen ist das für die Ausbildung der hydrophilen Beschichtung verwendete hydrophile Material ausgewählt unter Cellulosen, wie z.B. Methylcellulose, Carboxymethylcellulose, Hydroxymethylcellulose und Hydroxypropylcellulose oder unter Salep Mannan, Guaran, Carobin, Stärke, Xanthan Gum, Carmellose, Hypromellose, Macrogole, Gummi arabicum, Traganth, Karaya-Gummi, Kollagen, Fibrin, Elastin, Chitosan, Hyaluronsäure, Alginaten, Gelatine, Chitin, Heparin und Dextran Pektin, Carragen, Agar und Agarose.

Bei besonderen Ausführungsformen besteht die hydrophile Schicht aus einem hydrophilen Polymermaterial, das ausgewählt ist unter hydrophilen Homo- und Copolymeren von Acrylsäure, Salzen von Homo- und Copolymeren von Methacrylsäure, Salzen von Homo- und Copolymeren von Maleinsäure, Salzen von Homo- und Copolymeren von Fumarsäure, Salzen von Homo- und Copolymeren von Monomeren, die Sulfonsäuregruppen umfassen, Homo- und Copolymeren von Monomeren, die quaternäre Ammoniumsalze umfassen, und Gemischen und/oder Derivaten davon, mit der Maßgabe, dass die genannten Materialien den unter den genannten Bedingungen mit Wasser verlangten Kontaktwinkel ausbilden.

Das gewichtsmittlere Molekulargewicht $M_w$ der oben genannten Polymere liegt vorzugsweise im Bereich von 8.000 bis 5.000.000 g/mol, vorzugsweise im Bereich von 20.000 bis 3.000.000 g/mol und noch bevorzugter im Bereich von 200.000 bis 2.000.000 g/mol. In bestimmten Ausführungsformen sind die Polymerketten des jeweils eingesetzten Polymers miteinander vernetzt.

Zweckmäßigerweise ist die hydrophile Schicht - ebenso wie das Material der äußeren Schlauchschicht - elastisch. Vorzugsweise, besteht die hydrophile Schicht aus einem Elastomer.

Bei bestimmten Ausführungsformen ist die hydrophile Schicht zusätzlich dadurch gekennzeichnet, dass sie aus einem mit Wasser quellfähigem Material besteht. Vorzugsweise handelt es sich bei dem quellfähigen Material um ein Material, das bei 20°C pro 1 g quellfähigem Material wenigstens 1 g destilliertes Wasser aufnehmen kann. Bei bestimmten Ausführungsformen der Erfindung kann das quellfähige Material der hydrophilen Schicht wenigstens 10 g destilliertes Wasser oder gar wenigstens 100 g destilliertes Wasser aufnehmen.

Bei den Ausführungsformen, bei denen die hydrophile Schicht aus einem mit Wasser quellfähigem Material besteht, ist dieses Material vorzugsweise unter Materialien ausgewählt, die im gequollenen Zustand unter den genannten Bedingungen mit Wasser einen Kontaktwinkel θ < 30° ausbilden. Bei speziellen Ausführungsformen der Erfindung besteht die hydrophile Schicht aus einem Material, das im gequollenen Zustand unter den genannten Bedingungen mit Wasser einen Kontaktwinkel θ < 25° oder gar θ < 15°ausbildet.

Zur Bestimmung des Kontaktwinkels eines Schichtmaterials im gequollenen Zustand wird das Material vor der Kontaktwinkelmessung für 2 Minuten mit destilliertem Wasser benetzt, und auf der Oberfläche überstehendes Wasser wird danach und unmittelbar vor der Kontaktwinkelmessung mit Druckluft (6 bar, 1 Sekunde) abgeblasen.

Erfindungsgemäß besteht die hydrophile Schicht aus einem Material, das unter den genannten Bedingungen im gequollenen Zustand einen geringeren Kontaktwinkel θ mit Wasser ausbildet als die oben genannten herkömmlichen Füllschlauchmaterialien. Bei speziellen Ausführungsformen der Erfindung besteht die hydrophile Schicht aus einem Material, dessen Kontaktwinkel θ im gequollenen Zustand um wenigstens 15°, vorzugsweise wenigstens 20°, noch bevorzugter wenigstens 25° geringer ist als der Kontaktwinkel des für die äußere Schlauchschicht verwendeten herkömmlichen Füllschlauchmaterials.

Damit der Querschnitt des Lumens des Füllschlauchs möglichst groß bleibt, soll die Schichtdicke der hydrophilen Schicht möglichst gering sein. Vorzugsweise liegt die Schichtdicke der hydrophilen Schicht im Füllschlauch im trockenen, d.h. nicht gequollenen, Zustand im Bereich von 0,1 bis 5 μm, wobei unter trockenem Zustand hier der Zustand nach 72 Stunden bei 20°C ohne Kontakt mit flüssigem Wasser verstanden wird. Bei bestimmten Ausführungsformen der Erfindung ist die Schichtdicke der hydrophilen Schicht im trockenen Zustand im Bereich von 0,3 bis 3 μm.

Die Dicke der Beschichtung im mit Wasser gequollenen Zustand liegt vorzugsweise im Bereich von 10 bis 200 μm. Vorzugsweise ist die Dicke der Beschichtung im mit Wasser gequollenen Zustand weniger als 150 μm, noch bevorzugter weniger als 100 μm und besonders bevorzugt weniger als 50 μm. Zur Bestimmung der Dicke der Beschichtung im gequollenen Zustand wird das Material vor Messung für 2 Minuten mit destilliertem Wasser benetzt, und auf der Oberfläche überstehendes Wasser wird danach und unmittelbar vor der Messung mit Druckluft (6 bar, 1 Sekunde) abgeblasen.

Bei den Ausführungsformen, bei denen zwischen der äußeren Füllschlauchschicht und der inneren hydrophilen Schicht eine Verbindungsschicht als Haftvermittler vorgesehen ist, beträgt die Dicke dieser Verbindungsschicht vorzugsweise im Bereich von 10 nm bis 10 μm. Vorzugsweise ist die Dicke der Verbindungsschicht im mit Wasser gequollenen Zustand weniger als 75 μm, noch bevorzugter weniger als 50 μm und besonders bevorzugt weniger als 20 μm.

Bei bestimmten Ausführungsformen ist die Trachealbeatmungsvorrichtung der Erfindung dadurch gekennzeichnet, dass der auf der Innenseite mit einer hydrophilen Schicht ausgerüstete Füllschlauch im trockenen Zustand im Querschnitt einen Innendurchmesser von kleiner 1,2 mm aufweist. Bei speziellen Ausführungsformen kann der Innendurchmesser des Füllschlauchs im trockenen Zustand sogar kleiner oder gleich 0,8 mm oder gar kleiner oder gleich 0,6 mm betragen.

Bei bestimmten Ausführungsformen ist die Trachealbeatmungsvorrichtung der Erfindung dadurch gekennzeichnet, dass der auf der Innenseite mit einer hydrophilen Schicht ausgerüstete Füllschlauch im gequollenen Zustand im Querschnitt einen Innendurchmesser von größer oder gleich 0,3 mm aufweist.

Um kontrollieren zu können, ob der Cuff aufgeblasen ist, ist bei bestimmten Ausführungsformen der Erfindung ein Kontrollballon an den Füllschlauch angeschlossen, der in Fluidkontakt mit dem Lumen des Cuffs steht. Durch haptisches Befühlen des Kontrollballons kann der Druck grob abgeschätzt werden. Ein schlaffer Kontrollballon zeigt bereits optisch an, dass kein erhöhter Druck vorliegt. Ein prall gefüllter Kontrollballon zeigt dagegen optisch an, dass erhöhter Druck vorliegt.

Bei bestimmten Ausführungsformen der Erfindung ist am proximalen Ende des Füllschlauchs oder am proximalen Ende des Kontrollballons ein Füllventil angeschlossen, über das Luft oder ein anderes Gas durch den Füllschlauch in den Cuff eingespeist werden kann.

Über das Füllventil oder einen anderen geeigneten Anschluss ist eine Druckmesseinrichtung an den Füllschlauch bzw. an einen daran angeordneten Kontrollballon anschließbar, um hierüber den im Cuff vorliegenden Druck messen zu können. Zum Einstellen und Überprüfen des Cuffdrucks ist bei bestimmten Ausführungsformen ein entsprechend konfektioniertes Manometer an den Kontrollballon oder den Füllschlauch angeschlossen.

Je nach konkreter Ausführungsform ist an der Trachealbeatmungsvorrichtung ein passendes Verbindungsstück zur Verbindung mit einem Atemsystem vorgesehen und/oder eine Halsplatte als Anlagefläche am Hals des Patienten.

Von der vorliegenden Erfindung wird auch ein Verfahren zur Verbesserung der Druckmessung in der Manschette einer herkömmlichen Trachealbeatmungsvorrichtung umfasst, wobei die Trachealbeatmungsvorrichtung ein Kanülenrohr und eine um das Kanülenrohr herum angeordnete aufblasbare Manschette aufweist und wobei die Manschette mit einem Füllschlauch in Fluidkontakt ist. Erfindungsgemäß wird zur Verringerung des Durchflusswiderstandes die Aufbringung einer hydrophilen Schicht auf die Innenwand des Füllschlauchs vorgeschlagen.

Bei bestimmten Ausführungsformen erfolgt das Aufbringen der hydrophilen Schicht auf die Innenwand des Füllschlauchs unmittelbar bei der Herstellung der Trachealbeatmungsvorrichtung. Bei anderen Ausführungsformen erfolgt das Aufbringen der hydrophilen Schicht auf die Innenwand des Füllschlauchs erst zu einem späteren Zeitpunkt nach der Herstellung der Trachealbeatmungsvorrichtung und vorzugsweise kurz vor dessen Anwendung.

Das Aufbringen der hydrophilen Schicht erfolgt vorzugsweise, indem das hydrophile Beschichtungsmaterial, das die hydrophile Schicht ausbilden soll, in einer fließfähigen Form in das Lumen des herkömmlichen Füllschlauchs, welcher der späteren äußeren Schlauchschicht entspricht, eingeführt wird. Bei verschiedenen Ausführungsformen der Erfindung wird das hydrophile Beschichtungsmaterial entweder als flüssiges Material, als Suspension, als Lösung oder in Form eines Schaums oder Pulvers in den Füllschlauch eingeführt.

Die Umwandlung des in flüssiger Form, in gelöster Form, in suspendierter Form oder in Pulverform in den noch nicht beschichteten Füllschlauch eingebrachten Beschichtungsmaterials in eine feste, nicht lösliche Schicht, die fest mit der Innenwand des ursprünglichen Füllschlauchs verbunden ist, erfolgt entweder durch spontanes Aushärten, durch Trocknung, durch Anwendung erhöhter Temperatur oder durch Bestrahlung mit Licht. Bei bestimmten Ausführungsformen kommt es dabei zur Polymerisierung des Beschichtungsmaterials, zur Quervernetzung des Beschichtungsmaterials und/oder zu chemischen Verbindung des Beschichtungsmaterials mit dem Material der inneren Oberfläche des Füllschlauchs (Grafting) oder einer darauf vorgesehenen Verbindungsschicht.

**Ausführungsbeispiel**

Ein Füllschlauch aus PVC mit einem Innendurchmesser von 0,7 mm wurde mit Polyvinylpyrrolidon befüllt und für 30 Minuten auf 80°C erhitzt. Die Dicke der sich daraus ergebenden hydrophilen Beschichtung betrug 3 $\mu$m.

Die hydrophile Beschichtung führte dazu, dass das im Füllschlauch befindliche Wasser bereits bei sehr kleinen Druckdifferenzen durch den Füllschlauch befördert werden konnte. So konnte bei einem Versuch die Druckdifferenz, die notwendig war, um das Wasser durch den Füllschlauch zu drücken von 75 mbar auf 15 mbar reduziert werden.

Weitere Vorteile, Merkmale und Anwendungsmöglichkeiten der vorliegenden Erfindung werden anhand der folgenden Beschreibung einer Ausführungsform und den dazugehörigen Figuren deutlich, wobei gleiche Bezugszeichen auf gleiche Elemente verweisen. Es zeigen:

Figur 1: eine Trachealbeatmungsvorrichtung gemäß einer Ausführungsform der vorliegenden Erfindung und

Figur 2: sowohl einen Längs- als auch einen Querschnitt durch den Füllschlauch der Trachealbeatmungsvorrichtung aus Figur 1.

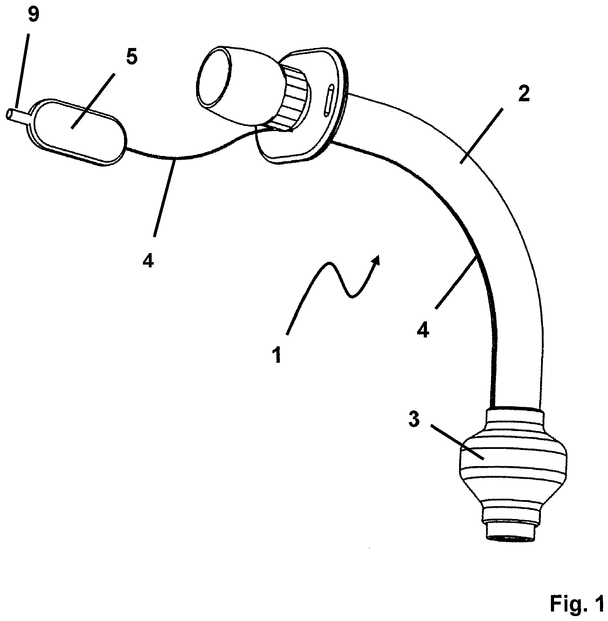

In der Figur 1 ist eine Trachealbeatmungsvorrichtung gemäß einer Ausführungsform der vorliegenden Erfindung gezeigt. Die hier dargestellte Trachealbeatmungsvorrichtung 1 ist eine Tracheostomiekanüle mit einem Kanülenrohr 2 und einer dicht mit der Außenseite des Kanülenrohrs 2 verbundenen Manschette (Cuff) 3. Der Cuff 3 ist in fluider Kommunikation über einen Füllschlauch 4 mit einem Kontrollballon 5 verbunden. Über das Füllventil 9 am Kontrollballon 5 kann der Cuff 3 durch den Füllschlauch 4 mit einem Gas befüllt bzw. der Cuffdruck reguliert werden.

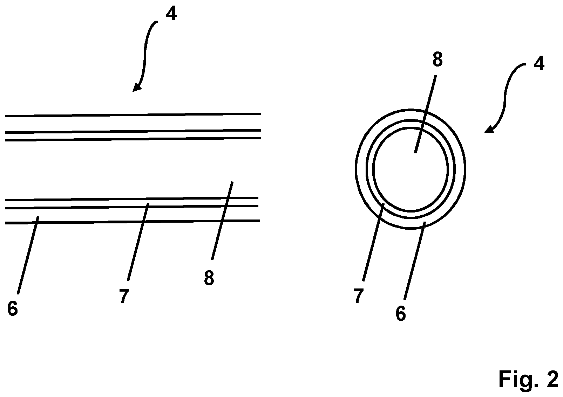

Wie in der Figur 2, die auf der linken Seite einen vergrößerten Längsschnitt durch den Füllschlauch 4 und auf der rechten Seite einen vergrößerten Querschnitt durch den Füllschlauch 4 zeigt, schematisch dargestellt ist, wird die innere Oberfläche des Füllschlauchs 4 von einer hydrophilen Schicht 7 gebildet, die auf der Innenseite der aus herkömmlichem Schlauchmaterial bestehenden äußeren Schlauchschicht 6 vorgesehen ist. Die hydrophile Schicht 7 liegt auf der Innenseite der äußeren Schlauchschicht 6 auf und ist mit dieser fest, d.h. nicht löslich, verbunden. Nach innen hin definiert die Oberfläche der hydrophilen Schicht 7 das Schlauchlumen 8.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird.

In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

**Bezugszeichenliste**

1 Trachealbeatmungsvorrichtung
2 Kanülenrohr
3 Manschette (Cuff)
4 Füllschlauch
5 Kontrollballon
6 äußere Schlauchschicht
7 hydrophile Schicht
8 Schlauchlumen
9 Füllventil

**Patentansprüche**

1. Trachealbeatmungsvorrichtung (1) mit einem Kanülenrohr (2), mit einem Füllschlauch (4) und einer um das Kanülenrohr (2) herum angeordneten aufblasbaren Manschette (3), wobei die Manschette (3) mit dem Füllschlauch (4) in Fluidkontakt ist, **dadurch gekennzeichnet, dass** die innere Oberfläche des Füllschlauchs (4) von einer festen hydrophilen Schicht (7) gebildet wird.

2. Trachealbeatmungsvorrichtung (1) nach Anspruch 1, **dadurch gekennzeichnet, dass** die hydrophile Schicht (7) aus einem hydrophilen Schichtmaterial besteht, das gemessen nach der Methode des liegenden Tropfens und berechnet nach der Youngschen Gleichung mit destilliertem Wasser bei 20°C einen Kontaktwinkel $\theta < 80°$ ausbildet.

3. Trachealbeatmungsvorrichtung (1) nach Anspruch 1 oder 2, **dadurch gekennzeichnet, dass** die hydrophile Schicht (7) die innere Oberfläche einer äußeren Füllschlauchschicht (6) flächig überzieht und fest mit dieser verbunden ist.

4. Trachealbeatmungsvorrichtung (1) nach einem der Ansprüche 1 bis 3, **dadurch gekennzeichnet, dass** die hydrophile Schicht (7) aus einem hydrophilen Polymermaterial besteht, das ausgewählt ist unter hydrophilen Poly(lactamen), Polyurethanen, Polyvinylalkohol, Polyvinylethern, Copolymeren auf Maleinsäureanhydridbasis, Polyestern, Vinylaminen, Polyethyleniminen, Polyethylenoxiden, Polypropylenoxiden, Poly(carbonsäuren), Polyamiden, Polyanhydriden, Polyphosphazenen, Polypeptiden, Polysacchariden, Polyestern, Oligonucleotiden, Polyvinylpyrrolidon, Polyvinylpyrrolidon-Copolymer, Polyvinylpolypyrrolidon, Polylactiden, Polyglycoliden und Polycaprolactonen.

5. Trachealbeatmungsvorrichtung (1) nach einem der Ansprüche 1 bis 4, **dadurch gekennzeichnet, dass** die hydrophile Schicht (7) aus einem hydrophilen Polymermaterial besteht, das ausgewählt ist unter hydrophilen Homo- und Copolymeren von Acrylsäure, Salzen von Homo- und Copolymeren von Methacrylsäure, Salzen von Homo- und Copolymeren von Maleinsäure, Salzen von Homo- und Copolymeren von Fumarsäure, Salzen von Homo- und Copolymeren von Monomeren, die Sulfonsäuregruppen umfassen, Homo- und Copolymeren von Monomeren, die quaternäre Ammoniumsalze umfassen, und Gemischen und/oder Derivaten davon.

6. Trachealbeatmungsvorrichtung (1) nach einem der Ansprüche 1 bis 5, **dadurch gekennzeichnet, dass** die hydrophile Schicht (7) aus einem mit Wasser quellfähigem Material besteht.

7. Trachealbeatmungsvorrichtung (1) nach einem der Ansprüche 1 bis 6, **dadurch gekennzeichnet, dass** die hydrophile Schicht (7) im trockenen Zustand eine Schichtdicke im Bereich von 0,1 bis 5 $\mu$m aufweist.

8. Trachealbeatmungsvorrichtung (1) nach einem der Ansprüche 1 bis 7, **dadurch gekennzeichnet, dass** die hydrophile Schicht (7) im mit Wasser gequollenen Zustand eine Schichtdicke im Bereich von 10 bis 200 $\mu$m aufweist.

9. Trachealbeatmungsvorrichtung (1) nach einem der Ansprüche 1 bis 8, **dadurch gekennzeichnet, dass** der Füllschlauch (4) im trockenen Zustand einen Innendurchmesser von kleiner 1,0 mm aufweist.

10. Trachealbeatmungsvorrichtung (1) nach einem der Ansprüche 1 bis 9, **dadurch gekennzeichnet, dass** der Füllschlauch (4) im mit Wasser gequollenen Zustand einen Innendurchmesser von größer 0,3 mm aufweist.

11. Verfahren zur Verbesserung der Druckmessung in der Manschette (3) einer Trachealbeatmungsvorrichtung (1) über einen Füllschlauch (4), wobei die Trachealbeatmungsvorrichtung (1) ein Kanülenrohr (2) und eine um das Kanülenrohr (2) herum angeordnete aufblasbare Manschette (3) aufweist, wobei die Manschette (3) mit einem Füllschlauch (4) in Fluidkontakt ist, **gekennzeichnet durch** Aufbringen einer hydrophilen Schicht (5) auf die Innenwand des Füllschlauchs (4).

12. Verfahren nach Anspruch 11, **dadurch gekennzeichnet, dass** das Aufbringen der hydrophilen Schicht (7) auf die Innenwand des Füllschlauchs (4) unmittelbar bei der Herstellung der Trachealbeatmungsvorrichtung (1) oder erst zu einem späteren Zeitpunkt nach der Herstellung der Trachealbeatmungsvorrichtung (1) und vor dessen Anwendung ausgeführt wird.

13. Verfahren nach Anspruch 11 oder 12 **dadurch gekennzeichnet, dass** das Aufbringen der hydrophilen Schicht erfolgt, indem ein hydrophiles Beschichtungsmaterial in einer fließfähigen Form in das Lumen des Füllschlauchs (4) eingeführt wird.

14. Verfahren nach Anspruch 13, **dadurch gekennzeichnet, dass** das Beschichtungsmaterial in flüssiger Form, in gelöster Form, in suspendierter Form oder in Pulverform in den Füllschlauch (4) eingeführt wird.

15. Verfahren nach einem der Ansprüche 13 und 14 **dadurch gekennzeichnet, dass** das in den Füllschlauch eingeführte Beschichtungsmaterial durch spontanes Aushärten, durch Trocknung, durch Anwendung erhöhter Temperatur und/oder durch Bestrahlung mit Licht in eine feste Beschichtung auf der Innenwand der äußeren Schlauchschicht umgewandelt wird.

## Claims

1. Tracheal ventilation device (1) having a cannula (2), having a filling tube (4) and an inflatable cuff (3) disposed around the cannula (2), the cuff (3) being in fluid contact with the filling tube (4), **characterized in that** the internal surface of the filling tube (4) is formed by a solid hydrophilic layer (7).

2. Tracheal ventilation device (1) according to Claim 1, **characterized in that** the hydrophilic layer (7) consists of a hydrophilic layer material which, measured by the sessile drop method and calculated by Young's equation, forms a contact angle θ < 80° with distilled water at 20°C.

3. Tracheal ventilation device (1) according to Claim 1 or 2, **characterized in that** the hydrophilic layer (7) surface-coats the internal surface of an external filling tube layer (6) and is firmly joined to said layer.

4. Tracheal ventilation device (1) according to any of Claims 1 to 3, **characterized in that** the hydrophilic layer (7) consists of a hydrophilic polymer material selected from hydrophilic poly(lactams), polyurethanes, polyvinyl alcohol, polyvinyl ethers, copolymers based on maleic anhydride, polyesters, vinylamines, polyethyleneimines, polyethylene oxides, polypropylene oxides, poly(carboxylic acids), polyamides, polyanhydrides, polyphosphazenes, polypeptides, polysaccharides, polyesters, oligonucleotides, polyvinylpyrrolidone, polyvinylpyrrolidone copolymer, polyvinylpolypyrrolidone, polylactides, polyglycolides and polycaprolactones.

5. Tracheal ventilation device (1) according to any of Claims 1 to 4, **characterized in that** the hydrophilic layer (7) consists of a hydrophilic polymer material selected from hydrophilic homo- and copolymers of acrylic acid, salts of homo- and copolymers of methacrylic acid, salts of homo- and copolymers of maleic acid, salts of homo- and copolymers of fumaric acid, salts of homo- and copolymers of monomers comprising sulfonic acid groups, homo- and copolymers of monomers comprising quaternary ammonium salts, and mixtures and/or derivatives thereof.

6. Tracheal ventilation device (1) according to any of Claims 1 to 5, **characterized in that** the hydrophilic layer (7) consists of a material swellable with water.

7. Tracheal ventilation device (1) according to any of Claims 1 to 6, **characterized in that** the hydrophilic layer (7) in the dry state has a layer thickness in the range from 0.1 to 5 μm.

8. Tracheal ventilation device (1) according to any of Claims 1 to 7, **characterized in that** the hydrophilic layer (7) in the state swollen with water has a layer thickness in the range from 10 to 200 μm.

9. Tracheal ventilation device (1) according to any of Claims 1 to 8, **characterized in that** the filling tube (4) in the dry state has an internal diameter of less than 1.0 mm.

10. Tracheal ventilation device (1) according to any of Claims 1 to 9, **characterized in that** the filling tube (4) in the state swollen with water has an internal diameter of more than 0.3 mm.

11. Method for improving the measurement of pressure in the cuff (3) of a tracheal ventilation device (1) by way of a filling tube (4), the tracheal ventilation device (1) having a cannula (2) and an inflatable cuff (3) disposed around the cannula (2), the cuff (3) being in fluid contact with a filling tube (4), **characterized by** application of a hydrophilic layer (5) to the inside wall of the filling tube (4).

12. Method according to Claim 11, **characterized in that** the application of the hydrophilic layer (7) to the inside wall of the filling tube (4) is performed directly during the production of the tracheal ventilation device (1) or only at a later point in time after the production of the tracheal ventilation device (1) and before the use thereof.

13. Method according to Claim 11 or 12, **characterized in that** the application of the hydrophilic layer takes place by introduction of a hydrophilic coating material in a flowable form into the lumen of the filling tube (4) .

14. Method according to Claim 13, **characterized in that** the coating material is introduced in liquid form, in dissolved form, in suspended form or in powder form into the filling tube (4).

15. Method according to either of Claims 13 and 14, **characterized in that** the coating material introduced into the filling tube is converted into a solid coating on the inside wall of the external tube layer by spontaneous curing, by drying, by use of elevated temperature and/or by irradiation with light.

## Revendications

1. Dispositif de ventilation trachéale (1) comportant une canule (2), un tuyau de gonflage (4) et un ballonnet gonflable (3), disposé autour de la canule (2), le ballonnet (3) étant en contact fluidique avec le tuyau de remplissage (4), **caractérisé en ce que** la surface intérieure du tuyau de remplissage (4) est formée d'une couche hydrophile solide (7).

**2.** Dispositif de ventilation trachéale (1) selon la revendication 1, **caractérisé en ce que** la couche hydrophile (7) est constituée d'un matériau en couche hydrophile, qui, la mesure étant effectuée par la méthode de la goutte sessile, et le calcul utilisant l'équation de Young, forme avec l'eau distillée à 20 °C un angle de contact θ < 80°.

**3.** Dispositif de ventilation trachéale (1) selon la revendication 1 ou 2, **caractérisé en ce que** la couche hydrophile (7) recouvre en pleine surface la surface intérieure d'une couche extérieure (6) du tuyau de remplissage, et y est fermement assemblée.

**4.** Dispositif de ventilation trachéale (1) selon l'une des revendications 1 à 3, **caractérisé en ce que** la couche hydrophile (7) est constituée d'un matériau polymère hydrophile qui est choisi parmi les poly(lactames) hydrophiles, les polyuréthanes, le poly(alcool vinylique), les polyvinyléthers, les copolymères à base d'anhydride maléique, les polyesters, les vinylamines, les polyéthylèneimines, les poly(oxydes d'éthylène), les poly(oxydes de propylène), les poly(acides carboxyliques), les polyamides, les polyanhydrides, les polyphosphazènes, les polypeptides, les polysaccharides, les polyesters, les oligonucléotides, la polyvinylpyrrolidone, un copolymère de polyvinylpyrrolidone, la polyvinylpolypyrrolidone, les polylactides, les polyglycolides et les polycaprolactones.

**5.** Dispositif de ventilation trachéale (1) selon l'une des revendications 1 à 4, **caractérisé en ce que** la couche hydrophile (7) est constituée d'un matériau polymère hydrophile choisi parmi les homo- et copolymères hydrophiles de l'acide acrylique, les sels d'homo- et de copolymères de l'acide méthacrylique, les sels d'homo- et de copolymères de l'acide maléique, les sels d'homo- et de copolymères de l'acide fumarique, les sels d'homo- et de copolymères de monomères comprenant des groupes acide sulfonique, les homo- et copolymères de monomères comprenant des sels de l'ammonium quaternaire, et les mélanges et/ou dérivés de ceux-ci.

**6.** Dispositif de ventilation trachéale (1) selon l'une des revendications 1 à 5, **caractérisé en ce que** la couche hydrophile (7) est constituée d'un matériau gonflable à l'eau.

**7.** Dispositif de ventilation trachéale (1) selon l'une des revendications 1 à 6, **caractérisé en ce que** la couche hydrophile (7) présente à sec une épaisseur de couche dans la plage de 0,1 à 5 µm.

**8.** Dispositif de ventilation trachéale (1) selon l'une des revendications 1 à 7, **caractérisé en ce que** la couche hydrophile (7) présente à l'état gonflé à l'eau une épaisseur de couche dans la plage de 10 à 200 µm.

**9.** Dispositif de ventilation trachéale (1) selon l'une des revendications 1 à 8, **caractérisé en ce que** le tuyau de remplissage (4) présente à sec un diamètre intérieur inférieur à 1,0 mm.

**10.** Dispositif de ventilation trachéale (1) selon l'une des revendications 1 à 9, **caractérisé en ce que** le tuyau de remplissage (4) présente à l'état gonflé à l'eau un diamètre intérieur supérieur à 0,3 mm.

**11.** Procédé d'amélioration de la mesure de la pression dans le ballonnet (3) d'un dispositif de ventilation trachéale (1) par l'intermédiaire d'un tuyau de remplissage (4), dans lequel le dispositif de ventilation trachéale (1) comprend une canule (2) et un ballonnet gonflable (3), disposé autour de la canule, le ballonnet (3) étant en contact fluidique avec un tuyau de remplissage (4), **caractérisé par** l'application d'une couche hydrophile (5) sur la paroi intérieure du tuyau de remplissage (4).

**12.** Procédé selon la revendication 11, **caractérisé en ce que** l'application de la couche hydrophile (7) sur la paroi intérieure du tuyau de remplissage (4) est réalisée immédiatement lors de la fabrication du dispositif de ventilation trachéale (1) ou seulement à un instant ultérieur, après la fabrication du dispositif de ventilation trachéale (1) et avant son utilisation.

**13.** Procédé selon la revendication 11 ou 12, **caractérisé en ce que** l'application de la couche hydrophile a lieu par introduction, dans la lumière du tuyau de remplissage (4), d'un matériau de revêtement hydrophile sous forme fluide.

**14.** Procédé selon la revendication 13, **caractérisé en ce que** le matériau de revêtement est introduit dans le tuyau de remplissage (4) sous forme liquide, sous forme dissoute, sous forme en suspension ou sous forme de poudre.

**15.** Procédé selon l'une des revendications 13 et 14, **caractérisé en ce que** le matériau de revêtement introduit dans le tuyau de remplissage est, par durcissement spontané, par séchage, par utilisation d'une température élevée et/ou par irradiation d'une lumière, converti en un revêtement solide sur la paroi intérieure de la couche extérieure du tuyau.

**Fig. 1**

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1861152 B1
- US 2008078406 A1
- US 2011027334 A1